# FASCICULE DE BREVET EUROPEEN

(11) **EP 1 187 575 B1**
(45) Date de publication et mention de la délivrance du brevet: **18.06.2003**
(21) Numéro de dépôt: 00929623.7
(22) Date de dépôt: 17.05.2000
(51) Int. Cl.: A61F 2/06

(54) **PROCEDE D'OBTENTION D'UN STENT TUBULAIRE A FAIBLE RESISTANCE A L'ECRASEMENT**
VERFAHREN ZUR HERSTELLUNG EINES ROHRFÖRMIGEN STENTS MIT SCHWACHEM QUETSCHWIDERSTAND
MANUFACTURING PROCESS OF A TUBULAR STENT HAVING A SLIGHT RESISTANCE TO CRUSHING

(30) Priorité: 18.05.1999 FR 9906538
(43) Date de publication de la demande: 20.03.2002
(73) Titulaire: Med-Xcor, 01600 Trevoux (FR)
(72) Inventeur: LEFEBVRE, Jean-Marie, 59800 Lille (FR)
(74) Mandataire: Guerre, Dominique
(86) Numéro de dépôt international: FR0001333
(87) Numéro de publication internationale: WO00069366

(56) Documents cités:
- EP-A- 0 876 806
- EP-A- 0 884 029
- WO-A-97/33534
- WO-A-98/40035
- WO-A-99/15107
- FR-A- 2 764 794
- SEERUYS AND KUTRYK: "Handbook of coronary stents" 1998 , MARTIN DUNITZ , LONDON, GB XP002147990 page 181 -page 186

## Description

La présente invention concerne un procédé d'obtention d'une endoprothèse, dénommée habituellement « stent », qui est utilisée en chirurgie cardio-vasculaire pour être implantée dans un conduit vasculaire, notamment artère coronaire ou artère périphérique. Elle concerne plus particulièrement un procédé d'obtention d'un stent réalisé à partir d'un tube en acier inoxydable dont la paroi a été découpée notamment par laser et qui est destinée à être implantée dans le conduit vasculaire au moyen d'un ballonnet gonflable.

Il est connu de traiter les maladies athéromateuses vasculaires, qui correspondent à des rétrécissements des artères , par des techniques de dilatation par ballonnets , dénommées angioplasties. Le rétrécissement d'une artère est provoqué par une infiltration de la paroi de ladite artère et par la formation d'un dépôt d'athérome à l'intérieur de la lumière de l'artère. Le ballonnet est introduit dans l'artère, est gonflé à une pression telle qu'il écrase le dépôt d'athérome. Les techniques d'angioplastie courramment utilisées souffrent d'un taux de récidive important, dû à un double phénomène, à savoir d'une part une rétractation élastique, communément appelée « recoil secondaire » de la zone dilatée et d'autre part une réaction inflammatoire appelée « prolifération intimale » de la partie interne de la paroi du vaisseau. En outre, une déchirure de la paroi interne de l'artère, dénommée « flap» peut entraîner une obstruction aigüe de l'artère , responsable d'accidents extrêmement graves.

Pour traiter et recoller les déchirures intimales et diminuer la resténose en évitant le recoil secondaire, il est connu d'implanter dans le conduit vasculaire , au niveau de la zone traitée par angioplastie, une endoprothèse, plus communément appelée « stent ». Un stent est un support métallique agissant comme tuteur une fois qu'il est introduit à l'intérieur de l'artère au niveau de la zone traitée. Dans la suite de la présente description, une telle endoprothèse sera désignée par le terme stent. On distingue trois types de stent : ceux fabriqués à partir d'un fil enroulé, ceux réalisés à partir d'un grillage et ceux réalisés à partir de tubes découpés notamment par laser. La présente invention se situe dans le domaine des stents réalisés à partir de tubes découpés qui sont conçus pour être dépliés au moyen d'un ballonnet gonflable lors de leur implantation dans le conduit vasculaire. A cet effet, le stent doit être serti sur le ballonnet , dans une position dite de repos, préalablement à l'introduction du ballonnet dans le conduit vasculaire . Une fois le stent acheminé jusqu'à sa zone d'implantation à l'intérieur du conduit vasculaire, il est déployé par expansion du ballonnet, en sorte d'être amené au contact de la paroi du conduit vasculaire.

L'opération préalable de sertissage du stent sur le ballonnet doit être réalisée en s'assurant que l'on n'abîme pas le stent ou le ballonnet, tout en veillant à obtenir une tenue parfaite du stent par rapport au ballonnet. Il est en effet primordial que le maintien en position du stent par rapport au ballonnet soit suffisant pour éviter les risques de déplacement du stent par rapport au ballonnet, lors de leur cheminement à I 'intérieur du conduit vasculaire à traiter, et en particulier à l'intérieur du cathéter de guidage pré-implanté dans le conduit vasculaire. Tout déplacement du stent peut d'une part faire obstacle à l'expansion du ballonnet et d'autre part occasionner une ouverture et une mise en place défectueuses du stent.

Au regard de l'opération de sertissage, deux cas de figures peuvent en pratique se présenter : le stent est vendu en étant déjà serti sur un ballonnet, ou le stent est vendu séparément sans être serti sur un ballonnet. Dans le premier cas , le sertissage du stent est effectué sur un ballonnet neuf, ayant une surface de révolutiuon parfaitement cylindrique , et est réalisé en écrasant le stent sur le ballonnet , de manière symétrique et régulière sur toute la longueur du stent et sur toute sa circonférence. Dans le second cas, le sertissage du stent est réalisé par le chirurgien , en règle générale sur un ballonnet ayant déjà été préalablement utilisé pour dilater la zone du conduit vasculaire devant être traitée. Le sertissage s'effectue dans ce cas sur un ballonnet qui a déjà été déployé ,et qui de ce fait comporte une membrane présentant des plissements asymétriques et qui ne forme plus un cylindre parfait.

Un stent doit présenter certaines caractéristiques dont les plus importantes sont la force radiale , la biocompatibilité , la flexibilité et la malléabilité.

La force radiale du stent doit être suffisante pour s'opposer à la rétraction élastique de la paroi du conduit vasculaire . Un stent fabriqué à partir d'un tube découpé présente l'avantage de présenter une force radiale plus importante que les deux autres types de stents (fil enroulé et grillage).

S'agissant de la biocompatibilité, le matériau utilisé pour fabriquer le stent doit évidemment être biocompatible et le moins thrombogène possible. S'agissant du matériau utilisé , à l'exception de quelques rares stents en Platinium, Tantalum ou Nitinol, la grande majorité des stents est aujourd'hui réalisée en acier inoxydable. La biocompatibilité , au regard notamment de l'aspect thrombogène , dépend également de l'état de surface du stent, c'est-à-dire principalement de la rugosité de la surface du stent. L'état de surface doit être le plus lisse possible.

La flexibilité du stent doit être suffisante pour permettre son acheminement dans le conduit vasculaire, qui peut être fortement sinueux, et en outre parfois calcifié. Pour améliorer la flexibilité des stents, il est largement répandu à ce jour de réaliser des stents tubulaires articulés , tels que ceux décrits par exemple dans la demande de brevet internationale WO96/33671. Un stent articulé se présente sous la forme d'une succession d'anneaux qui d'une part sont constitués de cellules ayant une forme géométrique déterminée et aptes à se déployer, et qui d'autre part sont reliés dans la direction longitudinale du stent par des connections , en sorte d'être articulés les uns par rapport aux autres.

Malgré l'utilisation des stents, le taux de resténose reste élevé. De nombreux facteurs peuvent être mis en cause: état préalable du vaisseau, traumatisme de la paroi lors de la dilatation et de la mise en place du stent, caractéristiques propres à l'individu... Pour limiter ce taux , on a déjà pensé à différentes solutions parmi lesquelles la mise en oeuvre de pressions plus faibles pour la dilatation du ballonnet ou encore l'utilisation de stents ayant une quantité de métal par unité de longueur relativement faible.

En particulier, le document WO 98/40035 décrit un stent qui peut être déployé sous de faibles pressions. Ce stent est de préférence réalisé par perforation d'un tube en métal à mémoire de forme. Lorsqu'il est déployé dans un conduit vasculaire, ce stent exerce une force radiale qui est faible du fait du matériau utilisé. Un tel stent présente de bonnes performances in situ en ce sens qu'il ne cause aucun dommage au conduit vasculaire mais il peut s'écraser du fait de sa nature élastique lors de la rétraction du conduit vasculaire.

Le document FR-A-2764794 décrit un procédé de fabrication d'un stent tubulaire apte à se déployer et destiné à être implanté dans un conduit vasculaire. Ce stent comprend une succession d'anneaux constitués chacun de cellules et des connexions reliant lesdits anneaux dans la direction longitudinale du stent. Selon le procédé décrit dans ce document, on part d'un tube en acier inoxydable découpé par laser et soumis ensuite à un polissage électrolytique.

La présente invention concerne un procédé d'obtention d'un stent qui une fois implanté et déployé dans un conduit vasculaire exerce une faible pression sur la paroi de ce dernier. Plus précisément, la présente invention vise l'obtention d'un stent qui de manière connue est destiné à être implanté dans un conduit vasculaire ayant une force de rétraction élastique donnée, en étant déployé sous l'effet de l'expansion du ballonnet. De manière caractéristique, le stent est obtenu par découpe d'un tube en acier inoxydable et sa résistance à l'écrasement, une fois implanté et déployé dans le conduit vasculaire est sensiblement inférieure ou égale à deux fois la valeur de la rétraction élastique du conduit vasculaire et sensiblement supérieure ou égale à 1,2 fois la valeur de la rétraction élastique du conduit vasculaire.

Le demandeur a en effet mis en évidence qu'un tel stent s'avérait être extrêmement performant tant du point de vue de l'irritation du conduit vasculaire dans lequel il est implanté que du maintien de la paroi de ce dernier. En particulier, bien qu'exerçant une faible pression sur la paroi du conduit vasculaire, le stent de l'invention étant en acier inoxydable, il est parfaitement biocompatible et ne s'écrase pas sous l'effet des faibles contractions du conduit vasculaire dans lequel il est implanté comme c'est le cas du stent décrit dans le document WO 98/40035.

Lorsque le conduit vasculaire est une artère coronaire, la résistance à l'écrasement du stent déployé est, de préférence, de l'ordre de 0,2 10⁵ Pa.

De même, lorsque le conduit vasculaire est une artère périphérique, la résistance à l'écrasement du stent déployé est de préférence de l'ordre de 0,5 10⁵ Pa.

En d'autres termes, le stent obtenu selon le procédé de l'invention a une résistance à l'écrasement qui est légèrement supérieure aux forces de recoil et donc aux valeurs physiologiques du conduit vasculaire dans lequel il est implanté.

Ainsi, selon les dispositions particulières de l'invention , la force exercée par le stent contre la paroi interne du conduit vasculaire est déterminée en sorte de réduire les risques de réaction contraire du conduit vasculaire susceptible de participer à l'accroissement du taux de resténose.

La diminution de la force radiale pour arriver aux valeurs souhaitées dans le cadre de la présente invention est obtenue en diminuant corrélativement la quantité de métal mis en oeuvre, notamment en réduisant l'épaisseur du tube métallique dans lequel le stent est découpé.

Dans une variante préférée de réalisation, la diminution de la quantité de métal se combine à l'augmentation de la flexibilité. Selon cette variante, le stent se présente sous la forme d'une succession de segments annulaires comportant des cellules et articulés par des connexions. De manière caractéristique , le nombre de connexions entre deux segments annulaires est de préférence d'une connexion, voire éventuellement deux connexions.

De préférence dans le cas d'un stent destiné à être implanté dans une artère coronaire, la longueur, en projection sur l'axe longitudinal du stent, d'un segment annulaire est sensiblement égale à 1,4 mm, le nombre de connexions est égal à 1 et les segments annulaires sont constitués de 7 cellules. Dans ce cas, l'épaisseur du stent est de préférence sensiblement égale à 0,07 mm.

De même, dans le cas d'un stent destiné à être implanté dans une artère périphérique, la longueur en projection sur l'axe longitudinal du stent, d'un segment annulaire est sensiblement égale à 3,9 mm, le nombre de connexions est égal à 1 et les segments annulaires sont constitués de 6 cellules. Dans ce cas, l'épaisseur du stent est de préférence sensiblement égale à 0,16 mm.

De préférence , dans ce cas, les connexions successives sont décalées angulairement par rapport à l'axe longitudinal du stent pour former un trajet en hélice.

Cette disposition particulière vise , tout en améliorant la flexibilité du stent , à préserver l'homogénité de sa structure lorsque le stent est amené à être recourbé lors de son implantation dans le conduit vasculaire.

La longueur en projection sur l'axe longitudinal de chaque connexion est comprise entre 0,1 mm et le tiers et de préférence le quart de la longueur d'un segment annulaire.

De manière à améliorer le maintien en position du stent sur le ballonnet lors de l'introduction de celui-ci dans le conduit vasculaire, chaque segment annulaire se présente sous la forme d'un serpentin constitué par une succession de bras reliés deux à deux par une zone d'articulation, deux bras reliés par une zone d'articulation définissant une cellule, chaque bras d'un segment donné étant disposé obliquement par rapport à l'axe longitudinal du stent , selon un angle supérieur à 20°, de préférence compris entre 20 et 30°.

Pour augmenter encore cette propriété , avantageusement les bras de deux segments annulaires successifs ont une obliquité d'angle inverse.

L'articulation entre deux segments annulaires se fait dans ce cas par une ou deux connexions reliant deux zones d'articulation , chaque connexion étant courbe en sorte de prolonger l'obliquité des montants correspondants.

Dans un mode particulier de réalisation, les zones d'articulation de deux segments annulaires adjacents sont décalées en sorte que la direction générale de la connexion correspondante soit oblique.

La diminution de la quantité de métal mise en oeuvre pour la fabrication du stent présente comme inconvénient de rendre le stent faiblement opaque. Etant donné que le suivi de la progression du stent dans le conduit vasculaire est réalisé par méthode radiographique , il est nécessaire que le stent puisse être décelé précisément. S'il est faiblement opaque, celui-ci peut être une gêne pour la précision de son positionnement dans le conduit vasculaire. Pour compenser ce déficit d'opacité, il est connu de mettre en place des marqueurs radio-opaques aux extrémités des stents, ces marqueurs étant dans un métal dont la masse atomique est élevée et qui est biocompatible , notamment platine ou or.

Cependant le contact entre les deux métaux constitutifs d'une part du stent et d'autre part du marqueur radio-opaque est à l'origine d'un effet pile lorsque le stent est en contact du milieu physiologique, qui joue le rôle d'électrolyte. Cet effet peut se traduire par une corrosion de l'acier inoxydable dont est constitué le stent.

Le procédé d'obtention selon l'invention peut être adapté afin de proposer un stent équipé de marqueurs radio-opaques qui diminue l'éventualité d'une corrosion par effet pile.

De manière caractéristique , chaque marqueur radio-opaque est solidarisé au stent sous la forme d'une pièce recourbée et fermée autour d'un segment annulaire. Cette pièce recourbée n'est pas fixée définitivement au stent, comme cela peut être le cas d'un sertissage sur une zone évidée du stent prévue à cet effet. Dans le cas présent , la surface de contact entre le stent et le marqueur radio-opaque est beaucoup plus faible, ce qui limite l'effet pile susceptible d'entraîner la corrosion du stent.

Chaque marqueur est formé à partir d'un ruban qui subit une opération de découpe , pliage et polissage par tonnelage avant d'être clipsé sur le segment annulaire, formant l'extrémité du stent.

Avantageusement la pièce formant le marqueur radio-opaque comporte un revêtement de protection, apte à diminuer son potentiel électrique.

Dans un autre mode de réalisation, c'est le segment annulaire ou tout au moins la partie du segment annulaire destinée à recevoir le marqueur radio-opaque qui comporte un revêtement de protection apte à diminuer son potentiel électrique.

La présente invention sera mieux comprise à la lecture de la description qui va être faite d'un exemple préféré de réalisation d'un stent obtenu par découpe d'un tube en acier inoxydable à très faible force radiale et à flexibilité améliorée, illustré par le dessin annexé dans lequel :
- les figures 1 et 2 sont des représentations schématiques à plat de deux variantes de réalisation du stent, à l'état replié ; et

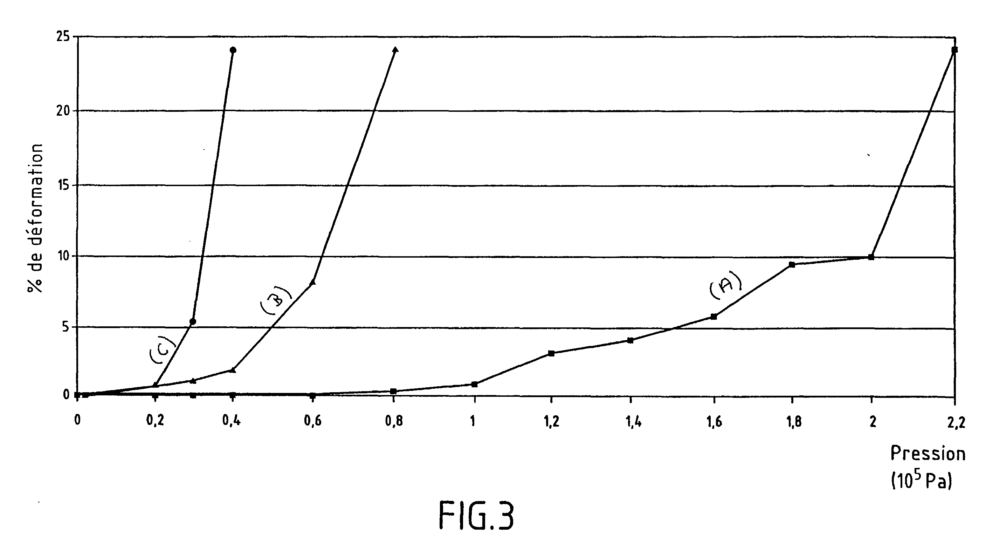

- la figure 3 est une courbe montrant l'écrasement sous contrainte comparativement du stent de l'invention et de deux autres stents connus.

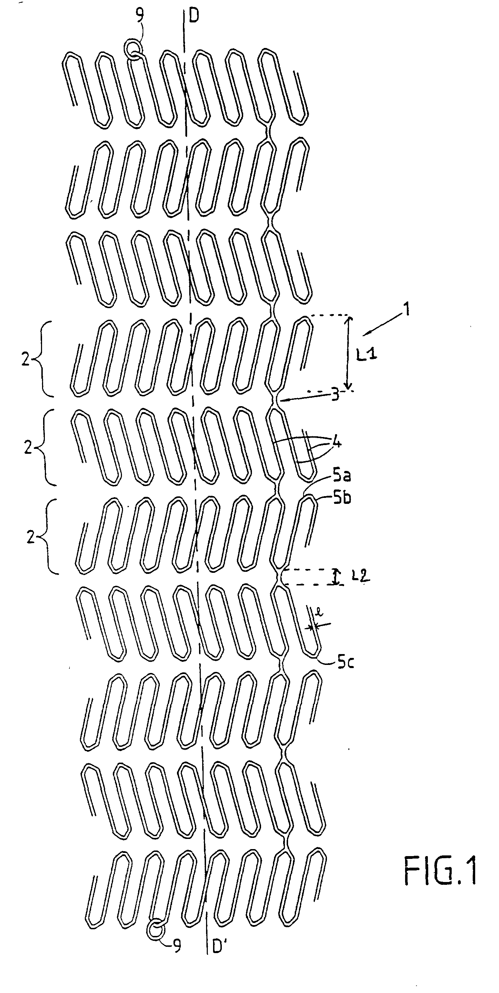

Le stent 1, selon la première variante, est obtenu par découpe au laser d'un tube en sorte de lui donner une configuration géométrique prédéterminée telle qu'illustrée à la figure 1. Ce tube découpé subit des opérations successives visant à le rendre utilisable pour l'application concernée. Il s'agit en premier lieu d'une opération de nettoyage par polissage électrolytique visant à enlever les bavures de découpe et les résidus de la carbonisation ; il s'agit ensuite d'un traitement thermique d'hypertrempe avec une première étape de cuisson du tube dans un four sous vide suivi d'une seconde étape de refroidissement rapide du tube au moyen d'azote liquide, ceci afin de diminuer la dureté de l'acier inoxydable. Le tube subit enfin un second traitement de polissage électrolytique en vue de réduire son épaisseur.

L'épaisseur recherchée pour le stent de l'invention est comprise entre O,06 mm et O,1 mm.

Le stent 1 est constitué d'une succession de segments annulaires de longueur L1 en projection selon l'axe longitudinal DD', et reliés dans la direction de l'axe longitudinal DD' du stent 1 par des connexions courbes 3 de longueur L2, en projection sur l'axe longitudinal DD'. Chaque segment annulaire 2 forme un serpentin apte à se déployer et est constitué de successions de bras 4 reliés deux à deux par une zone d'articulation 5.

Lorsque le stent 1 est au repos , les bras 5 de chaque segment 2 sont sensiblement parallèles et orientés transversalement à l'axe longitudinal DD' selon un angle α compris entre 20 et 30°. Plus particulièrement, les angles α de deux segments annulaires adjacents sont de valeurs opposées.

Sur la figure 1 , deux segments annulaires 2 adjacents sont reliés par une seule connexion 3 qui s'étend entre deux zones d'articulation 5 en regard l'une de l'autre. Chaque zone d'articulation 5 est constituée par deux montants en V 5a, 5b, la connexion étant reliée à la pointe 5c du V.

Dans la première variante de la figure 1, chaque connexion est courbe , avec une concavité qui est telle que la connexion 3 prolonge sensiblement deux montants 5c des zones d'articulation 5 en regard l'une de l'autre.

Dans cette même première variante, toutes les connexions 3 du stent sont sensiblement alignées , dans la même direction longitudinale par rapport à l'axe DD'.

D'autres modes de réalisation sont envisageables, notamment en vue de conférer une plus grande homogénéité de structure au stent 1 lors de sa déformation dans le cas où le conduit vasculaire dans lequel il est implanté n'est pas rectiligne mais courbe. Dans un mode préféré de réalisation, non représenté, les connexions 3 successives sont décalées angulairement par rapport à l'axe longitudinal DD' de manière à ce que les connexions successives se présentent en forme d'hélice sur la périphérie du stent.

Dans un mode précis de réalisation, le stent 1 est réalisé en acier inox 316 L , il a une longueur totale de l'ordre de 17mm. L'angle α d'inclinaison des bras 4 de chaque segment annulaire 2 est de 20° par rapport à l'axe longitudinal DD'. L'épaisseur du tube , après découpe et finition , est de 0,07 mm , tandis que la largeur I du serpentin constituant chaque segment annulaire et chaque connexion 3 est de O,1 mm. La longueur L1 en projection sur l'axe longitudinal DD' de chaque segment annulaire 2 est de 1,39mm et la longueur L2 en projection sur l'axe longitudinal DD' de chaque connexion 3 est de O,34mm. Le nombre de segments annulaires 2 est de dix et le nombre de connexions 3 est de neuf. Dans le cas d'un stent destiné à être implanté dans une artère coronaire, la largeur I du serpentin est sensiblement égale à O,1mm. Dans le cas d'un stent destiné à être implanté dans une artère périphérique, la largeur I est de préférence sensiblement égale à O,22mm.

Dans l'exemple ci-dessus, la longueur L2, d'une connexion 3 est légèrement inférieure au quart de la longueur L1 d'un segment annulaire 2.

On sait mesurer l'écrasement sous contrainte d'un stent , à l'état déployé dans des conditions normales d'implantation dans un conduit vasculaire , pour des pressions progressivement croissantes.

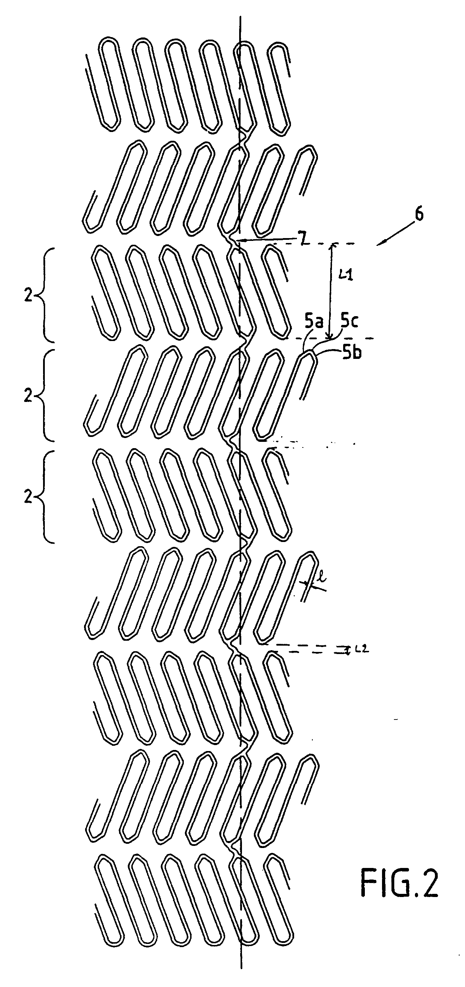

On a reporté sur la courbe de la figure 2 des valeurs obtenues pour le stent 1 obtenu selon le procédé d'obtention de l'invention et pour deux autres stents connus ayant des valeurs d'écrasement extrêmes. La courbe donne en ordonnée le pourcentage de déformation et en abscisse la pression exercée sur le stent, exprimée en bars c'est-à-dire en 10⁵ Pa.

Pour le stent connu (courbe A) ayant la plus grande résistance à l'écrasement, il faut une pression de l'ordre de 1,6 bar pour obtenir une déformation supérieure à 5%, tandis qu'il faut une pression de l'ordre de 0,5 bar pour obtenir la même déformation avec le stent connu le moins résistant à l'écrasement (courbe B).

Par contre, s'agissant du stent obtenu selon le procédé d'obtention de l'invention (courbe C), cette même déformation, supérieure à 5% est obtenue avec une pression de l'ordre de 0,3 bar.

La caractéristique de l'invention est que la force radiale exercée par le stent soit légèrement supérieure à la force de rétraction naturelle du conduit vasculaire dans lequel il est implanté.

Dans le cas d'une artère coronaire, cette force de rétraction naturelle, ou force de recoil est de l'ordre de 0,1 à 0,2 bar. Le stent de l'invention est déterminé pour que sa force radiale ou résistance à l'écrasement soit , dans ce cas, de l'ordre de 0,2 bar ou légèrement supérieure à cette valeur, comprise entre 0,2 et 0,3 bar.

La seconde variante de réalisation qui est illustrée à la figure 2 est identique à la première variante en ce qui concerne la configuration de chaque segment annulaire. Par contre elle diffère principalement par le décalage des zones d'articulation de deux segments annulaires adjacents , ce qui entraîne corrélativement une différence quant à la forme de chaque connexion.

Dans le stent 6, selon cette seconde variante , illustrée à la figure 2, chaque connexion 7 a une forme sensiblement en S avec deux points d'inflexion avec les extrémités de ladite connexion étant reliées au sommet 5c des deux bras 5a, 5b en forme de V constituant les zones d'articulation 5 du segment annulaire 2.

Dans cette seconde variante , la longueur L1 en projection sur l'axe longitudinal DD' de chaque segment annulaire est de 3,9 mm, la longueur L2 en projection sur l'axe longitudinal DD' de chaque connexion 8 de O,36mm , le nombre de segments annulaires 2 est de neuf , le nombre de connexions 7 est de huit et la longueur totale du stent est de 37,98mm.

La diminution de la quantité de métal dans le stent obtenu selon le procédé d'obtention de l'invention rend quasiment obligatoire la mise en oeuvre d'un marqueur radio-opaque aux deux extrémités du stent.

Selon une configuration particulière chaque marqueur est un cavalier formé à partir d'un ruban d'or ou de platine , qui subit une opération de découpe, pliage et polissage par tonnelage avant d'être refermé sur une zone d'articulation 5 du segment annulaire 2' d'extrémité. Il n'y a donc pas fixation proprement dite du marqueur radio-opaque sur le stent . Le cavalier 9 n'est en contact que sur une surface réduite avec la zone d'articulation correspondante, ce qui diminue au maximum les risques de corrosion par effet de pile lorsque le stent séjourne dans le conduit vasculaire et que les deux métaux (acier inoxydable et platine ou or) sont dans le liquide physiologique faisant office d'électrolyte.

Si l'on veut encore diminuer ce risque minime de corrosion , il est possible de prévoir sur la surface extérieure du cavalier 9 un revêtement de protection apte à diminuer le potentiel électrique dudit cavalier 9. Ou encore il est possible d'appliquer ce revêtement de protection non plus sur le cavalier 9 mais sur la partie du stent venant en contact avec le cavalier 9.

## Revendications

1. Procédé d'obtention d'un stent tubulaire apte à se déployer, destiné à être implanté dans un conduit vasculaire, ledit stent comprenant une succession d'anneaux constitués chacun de cellules, et des connections reliant lesdits anneaux dans la direction longitudinale dudit stent, procédé selon lequel :
(a) on part d'un tube en acier inoxydable, n'ayant aucune mémoire de forme, et on découpe ledit tube par laser
(b) on polit électrolytiquement une première fois ledit tube découpé, puis on le traite thermiquement par une hypertrempe comprenant une étape de cuisson sous vide puis une étape de refroidissement dudit tube, puis on le polit électrolytiquement une deuxième fois
**caractérisé en ce que** :
(1) on détermine la force de rétraction élastique du conduit vasculaire, auquel ledit stent est destiné
(2) le stent est obtenu en sorte qu'il présente, dans son état déployé une résistance à l'écrasement, inférieure ou égale à deux fois la valeur de ladite force de rétractation élastique, et supérieure ou égale à 1,2 fois la valeur de la dite force de rétraction élastique

## Claims

1. Method of obtaining a tubular stent which is able to deploy and is intended to be implanted in a vascular conduit, said stent comprising a succession of rings, each made up of cells, and connections joining said rings in the longitudinal direction of said stent, in which method:
(a) one starts with a tube of stainless steel having no shape memory, and said tube is cut out by laser,
(b) said cut-out tube is electropolished a first time, then subjected to a thermal treatment by rapid quenching, comprising a step of vacuum annealing of said tube, then a step of cooling said tube, then it is electropolished a second time,
**characterized in that**
(1) the elastic retraction force of the vascular conduit for which said stent is intended is determined,
(2) the stent is obtained in such a way that, in its deployed state, it has a resistance to crushing less than or equal to twice the value of said elastic retraction force, and greater than or equal to 1.2 times the value of said elastic retraction force.

## Patentansprüche

1. Verfahren zum Herstellen eines rohrförmigen Stents, der in der Lage ist, sich zu entfalten und dazu bestimmt ist, in einen vaskulären Leiter implantiert zu werden, wobei der Stent eine Abfolge von Ringen aufweist, die jeweils aus Zellen gebildet sind, und wobei Verbindungen die Ringe in Längsrichtung des Stents verbinden, wobei gemäß dem Verfahren:
(a) ausgehend von einem Rohr aus rostfreiem Stahl, der kein Formgedächtnis aufweist, das Rohr durch Lasern geschnitten wird
(b) das geschnittene Rohr ein erstes Mal elektrolytisch poliert, dann durch eine Überhärtung thermisch behandelt wird, die einen Schritt eines Vakuumbrennens und dann einen Schritt des Abkühlens des Rohrs umfasst, und es dann ein zweites Mal elektrolytisch poliert wird,
**dadurch gekennzeichnet, dass**:
(1) die elastische Retraktionskraft des vaskulären Leiters, für den der Stent vorgesehen ist, bestimmt wird,
(2) der Stent derart hergestellt wird, dass er in seinem entfalteten Zustand einen Stauchungswiderstand aufweist, der kleiner oder gleich dem zweifachen Wert der elastischen Retraktionskraft und größer oder gleich dem 1,2-fachen Wert der elastischen Retraktionskraft ist.
